# EUROPEAN PATENT SPECIFICATION

(11) **EP 2 921 206 B1**
(45) Date of publication and mention of the grant of the patent: **01.03.2017**
(21) Application number: 15152542.5
(22) Date of filing: 26.01.2015
(51) Int. Cl.: A61N 5/10, G21K 5/10

(54) **Neutron capture therapy system**
Neutroneneinfangtherapievorrichtung
Dispositif de thérapie par capture neutronique

(30) Priority: 18.03.2014 JP 2014054744
(43) Date of publication of application: 23.09.2015
(73) Proprietor: SUMITOMO HEAVY INDUSTRIES, LTD., Tokyo 141-6025 (JP)
(72) Inventor: Ilo, Itsushi, Ehime 792-8588 (JP)
(74) Representative: Carstens, Dirk Wilhelm

(56) References cited:
- EP-A1- 2 612 692
- US-A1- 2011 066 278

## Description

### BACKGROUND OF THE INVENTION

### Field of the Invention

The present disclosure relates to a neutron capture therapy system.

### Description of Related Art

JP 2007-289373 A discloses a radiation treatment facility including: a treatment room in which radiation therapy is performed; an irradiation nozzle for irradiating the inside of the treatment room with radiation rays; a preparation room in which preparation for treating a patient is performed; and a treatment table capable of being moved between the treatment room and the preparation room with a patient thereon. In the preparation room, positioning is performed such that a lesion area of a patient lying down on the treatment table is coincident with a position at which radiation rays are emitted. Next, the patient is moved to the treatment room together with the treatment table. Then, the lesion area of the patient is irradiated with radiation rays from the irradiation nozzle based on the positioning done in the preparation room. Accordingly, the operation in the treatment room is simplified, and therefore, it is possible to reduce the time for which one patient occupies the treatment room. Accordingly, it is possible to enhance utilization efficiency of the radiation treatment facility.

JP 2007-289373 A does not disclose any specific configuration with which the treatment table can accurately and precisely be moved between the preparation room and the treatment room.

As an example of a moving mechanism of the treatment table, it can be considered that a track such as a linear guide or a rail is built on a floor of the radiation treatment facility, and the treatment table is moved between the preparation room and the treatment room along the track. However, when providing the track on the floor surface or within a groove which is formed on the floor surface, there is a concern that a person (for example, a patient, a medical practitioner, or a health care provider) will stumble over the track protruding from the floor surface or the groove on the floor surface. For this reason, consideration of safety has been required.

US 2011/066278 A1 discloses a device for positioning a patient with respect to a radiation, the device being a polyarticulated robot including: at least one linear horizontal displacement rail, a connecting part capable of carrying out translations with respect to the linear rail and pivoting about an axis of rotation vertical to that linear rail, and a robotic arm connected to the connecting part, the robotic arm including a wrist with concurrent axes of rotation connected to a patient support.

EP 2 612 692 A1 discloses a neutron beam irradiation system that irradiates an irradiated body with a neutron beam. The system includes: a neutron beam generating section that is irradiated with a charged particle beam, thereby generating a neutron beam; an accommodation section that includes at least a moderator and accommodates the neutron beam generating section; a neutron beam converging section that converges the neutron beam which is irradiated to the irradiated body; a placement table on which the irradiated body is placed; and relative movement means that relatively moves the placement table so as to approach and be separated from the neutron beam generating section, the accommodation section, and the neutron beam converging section.

### SUMMARY OF THE INVENTION

Therefore, the present disclosure describes a neutron capture therapy system which can enhance safety.

According to the present invention, there is provided a neutron capture therapy system as set forth in claim 1. Preferred embodiments of the present invention may be gathered from the dependent claims.

In the neutron capture therapy system according to the present invention, the guide rail is provided in the recessed groove portion. For this reason, the guide rail does not protrude from the floor surface. Accordingly, there is no concern that a person will stumble over the guide rail protruding from the floor surface. In the neutron capture therapy system according to the aspect of the present disclosure, although the recessed groove portion is provided on the floor surface, the belt is provided in the recessed groove portion so as to cover the upper surface of the guide rail. In addition, the upper surface of the portion of the belt which is not lifted by the belt lifting portion constitutes a substantially identical surface to the floor surface. For this reason, the recessed groove portion is closed by the belt, and therefore, there is no concern that a person will stumble over the recessed groove portion on the floor surface. Accordingly, it is possible to enhance safety.

In the neutron capture therapy system according to the present invention, the belt lifting portion partially lifts the belt, which is positioned in the recessed groove portion, above the recessed groove portion. For this reason, at a site at which the belt is lifted by the belt lifting portion, the guide rail in the recessed groove portion is exposed to the outside. The treatment table slides on the portion of the guide rail, which is exposed to the outside, along the guide rail. Accordingly, the portion of the belt positioned below the treatment table is lifted by the belt lifting portion as the treatment table moves on the guide rail, and the remainder of the belt (portion of the belt which is not lifted by the belt lifting portion) is provided in the recessed groove portion and constitutes an identical surface to the floor surface. As a result, it is possible to move the treatment table along the guide rail while enhancing safety. Accordingly, it is possible to achieve both the movement of the treatment table and the safety thereof.

The system may further include a pair of support members which is positioned in the recessed groove portion and on both sides of the guide rail, and extends along the recessed groove portion, in which the portion of the belt which is not lifted above the recessed groove portion by the belt lifting portion is supported at three points including the upper surface of the guide rail and upper surfaces of the pair of support members. In this case, it is possible to reliably support the belt using the guide rail and the pair of support members.

In the system, the guide rail, the recessed groove portion, and the belt may extend from the inside of the irradiation room to the outside.

In the system, a recessed and projected surface on which recesses and projections are arranged in an extending direction of the belt may be formed on the surface of the belt on a side facing the recessed groove portion; a recessed and projected portion meshing with the recessed and projected surface of the belt is formed on the surface of the belt lifting portion; and the treatment table may further have a rail engagement portion which is engaged with the guide rail and is slidable along the guide rail, and a driving source which rotates the belt lifting portion. In this case, the power of a driving source can be transmitted to the belt from the belt lifting portion. Accordingly, it is possible to move the treatment table along the guide rail by rotating the belt lifting portion using the driving source.

In the system, the rail engagement portion may have a slider main body, and a rolling member which is accommodated in the slider main body and has a lubricant adhered to a surface thereof. In this case, the slider main body smoothly moves due to the rolling member and the lubricant. Accordingly, the lubricant is adhered to the surface of the guide rail. However, the belt covers the upper surface of the guide rail, and therefore, it is possible to prevent the lubricant from being adhered to the floor surface or the like.

According to the neutron capture therapy system according to the present disclosure, it is possible to enhance the safety.

### BRIEF DESCRIPTION OF THE DRAWINGS

Fig. 1 is a schematic view showing a neutron capture therapy system.
Fig. 2 is a view showing a configuration of the neutron capture therapy system.
Fig. 3 is a view showing an arrangement of the neutron capture therapy system.
Fig. 4 is a view showing a neutron beam output portion and its surroundings.
Fig. 5 is a perspective view showing a treatment table.
Fig. 6 is a cross-sectional view taken along line VI-VI of Fig. 5.
Fig. 7 is a cross-sectional view taken along line VII-VII of Fig. 6.
Fig. 8 is a cross-sectional view taken along line VIII-VIII of Fig. 6.
Fig. 9 is a perspective view showing a portion of a driving belt.

### DETAILED DESCRIPTION OF THE INVENTION

The embodiment of the present invention will be described with reference to the accompanying drawings. The following embodiment is an example for describing the present invention, and is not intended to limit the present invention to the following contents. In the description, identical elements or elements having the same functions are given the same reference numerals and the repeated description thereof will be omitted.

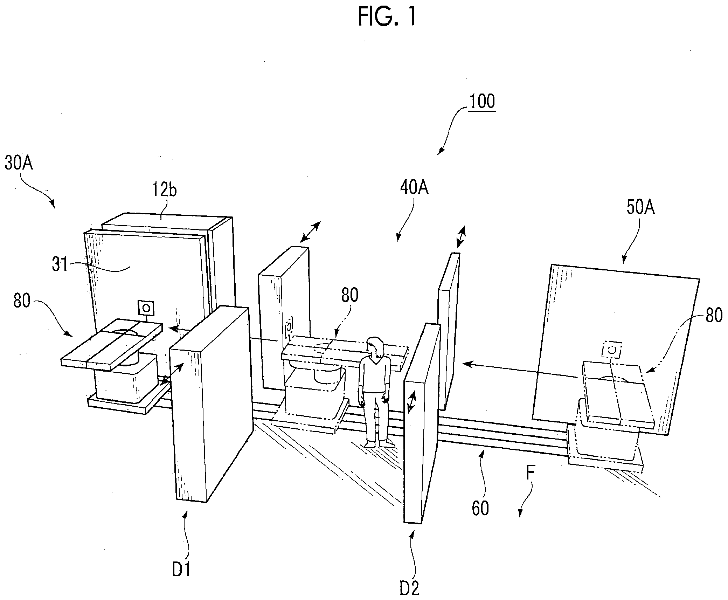

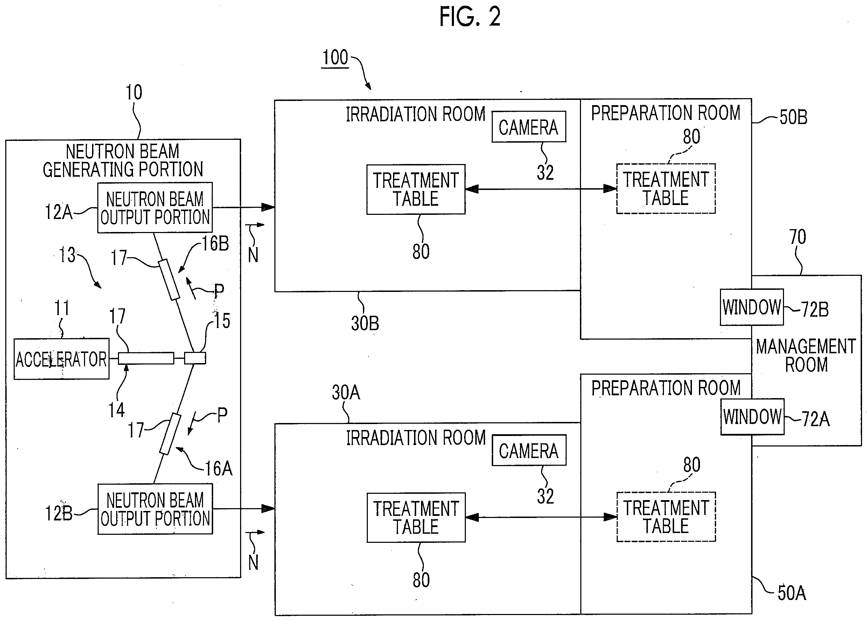

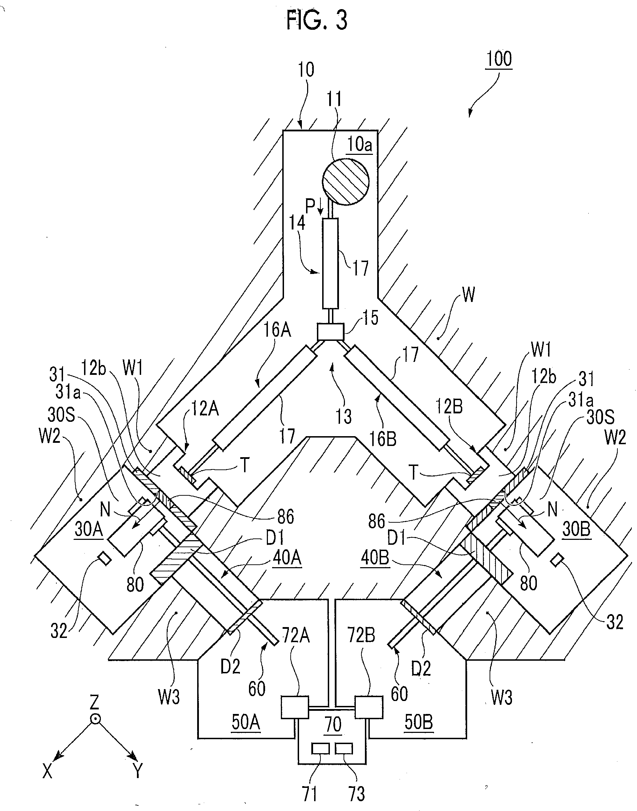

First, the entire configuration of a neutron capture therapy system 100 will be described with reference to Figs. 1 to 3. The neutron capture therapy system 100 is a system for treating cancer using boron neutron capture therapy (BNCT). The neutron capture therapy is a therapy for treating cancer by irradiating a patient (irradiation object), to which boron (¹⁰B) is administered, with a neutron beam. As shown in Fig. 1, the neutron capture therapy system 100 carries out neutron capture therapy including a step of performing a preparation operation, such as restraining a patient on a treatment table (movement body) 80 in a state where the patient is laid on the treatment table 80, in a preparation room 50A; a step of moving the treatment table 80 to a irradiation room 30A for each patient; and a step of irradiating the patient with a neutron beam in the irradiation room 30A. As shown in Figs. 2 and 3, the neutron capture therapy system 100 includes a neutron beam generating portion 10, irradiation rooms 30A and 30B, preparation rooms 50A and 50B, a track 60, a management room 70, and a treatment table 80. The configuration of each portion will be described below. Neutron Beam Generating Portion

The neutron beam generating portion 10 generates a neutron beam N in irradiation rooms 30A and 30B and irradiates a patient S with the neutron beam N. As shown in Figs. 2 and 3, the neutron beam generating portion 10 includes an accelerator 11 (for example, cyclotron), neutron beam output portions 12A and 12B which generate the neutron beam N from a charged particle beam P, and a beam transportation path 13 which transports the charged particle beam P to the neutron beam output portions 12A and 12B. The accelerator 11 and the beam transportation path 13 are disposed in a charged particle beam generating room 10a which forms a Y shape (refer to Fig. 3). The charged particle beam generating room 10a is a closed space which is covered with a concrete shielding wall W.

The accelerator 11 accelerates charged particles (for example, protons), and generates and emits the charged particle beam P (for example, proton beam). The accelerator 11 has the ability to generate a charged particle beam P with, for example, a beam radius of 40 mm and 60 kW (= 30 MeV x 2 mA).

The beam transportation path 13 selectively emits the charged particle beam P to any one of the neutron beam output portions 12A and 12B. The beam transportation path 13 has a first transporting portion 14 which is connected to the accelerator 11, a beam direction switcher 15 which switches the traveling direction of the charged particle beam P, a second transporting portion 16A for transporting the charged particle beam P to the neutron beam output portion 12A, and a third transporting portion 16B for transporting the charged particle beam P to the neutron beam output portion 12B. The second transporting portion 16A is connected to the beam direction switcher 15 and the neutron beam output portion 12A. The third transporting portion 16B is connected to the beam direction switcher 15 and the neutron beam output portion 12B. That is, the beam transportation path 13 is branched into the second transporting portion 16A and the third transporting portion 16B in the beam direction switcher 15.

The beam direction switcher 15 controls the traveling direction of the charged particle beam P using a switching electromagnet. The beam direction switcher 15 can lead the charged particle beam P to a beam dump (not shown) by making the charged particle beam stray from the regular track. The beam dump checks output of the charged particle beam P before treatment or the like. The neutron capture therapy system 100 may not include the beam dump. In this case, the beam direction switcher 15 is not connected to the beam dump.

Each of the first transporting portion 14, the second transporting portion 16A, and the third transporting portion 16B includes a beam adj ustment portion 17 for the charged particle beam P. The beam adjustment portion 17 includes a horizontal type steering unit and a horizontal/vertical type steering unit for adjusting the axis of the charged particle beam P; a quadrupole electromagnet for suppressing scattering of the charged particle beam P; and a four-direction slit for shaping the charged particle beam P. Each of the first transporting portion 14, the second transporting portion 16A, and the third transporting portion 16B may not include the beam adjustment portion 17.

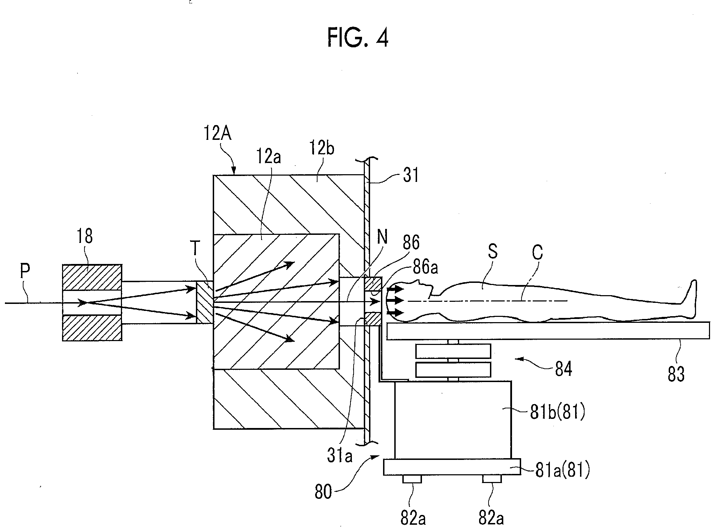

The second transporting portion 16A and the third transporting portion 16B may include a current monitor as necessary. The current monitor measures a current value (that is, electrical charge and irradiation dose rate) of the charged particle beam P with which the neutron beam output portions 12A and 12B are irradiated in real time. The second transporting portion 16A and the third transporting portion 16B may include a charged particle beam scanning portion 18 (refer to Fig. 4) as necessary. The charged particle beam scanning portion 18 scans the charged particle beam P and controls irradiation of a target T (refer to Fig. 4) with the charged particle beam P. The charged particle beam scanning portion 18 controls, for example, the position at which the target T is irradiated with the charged particle beam P.

As shown in Fig. 4, the neutron beam output portion 12A includes a target T for generating a neutron beam N, a moderator material 12a for reducing the speed of the neutron beam N, and a shielding body 12b. The moderator material 12a and the shielding body 12b constitute a moderator. The neutron beam output portion 12A and the neutron beam output portion 12B have the same configuration as each other. Therefore, in the present disclosure, the neutron beam output portion 12A will be described, but the description of the neutron beam output portion 12B will be omitted.

The target T generates a neutron beam N by being irradiated with a charged particle beam P. The target T is formed of, for example, beryllium (Be), and forms a circular shape with a diameter of 160 mm. The target is not limited to have a tabular shape (solid), and may be in other states such as a liquid state.

The moderator material 12a reduces the speed of the neutron beam N emitted from the target T. A neutron beam N, the speed of which is reduced by the moderator material 12a and which has a predetermined reduced energy is also called a therapeutic neutron beamN. The moderator material 12a may form, for example, a stacked structure in which a plurality of different materials are stacked. The material of the moderator material 12a can be appropriately selected in accordance with various conditions such as the energy of the charged particle beam P. For example, when the output from the accelerator 11 (refer to Fig. 2) is a proton beam with 30 MeV and a beryllium target is used as the target T, lead, iron, aluminum, or calcium fluoride may be used as the material of the moderator material 12a. When the output from the accelerator 11 is a proton beam with 11 MeV and a beryllium target is used as the target T, heavy water (D₂O) or lead fluoride may be used as the material of the moderator material 12a. When the output from the accelerator 11 is a proton beam with 2.8 MeV and a lithium target is used as the target T, Fluental (trade name; mixture of aluminum, aluminum fluoride, and lithium fluoride) may be used as the material of the moderator material 12a. When the output from the accelerator 11 is a proton beam with 50 MeV and a tungsten target is used as the target T, iron or Fluental may be used as the material of the moderator material 12a.

The shielding body 12b shields radiation rays such as a neutron beam N and a gamma ray which is generated due to the generation of the neutron beam N so as not to be discharged to the outside. At least a portion of the shielding body 12b is embedded in a wall W1 (refer to Fig. 3) partitioning the charged particle beam generating room 10a and the irradiation room 30A.

In the neutron beam output portion 12A, the target T is irradiated with a charged particle beam P which results in a neutron beam N being generated. The speed of the generated neutronbeamN is reduced by the moderator material 12a. A patient S on a treatment table 80 is irradiated with the neutron beam N emitted from the moderator material 12a through a collimator 86. The neutron beamN contains a fast neutron beam, anepithermal neutron beam, and a thermal neutron beam, and also accompanies a gamma ray. Among these, the thermal neutron beam mainly exhibits an effective therapeutic effect by being subjected to a nuclear reaction with boron which is introduced into a tumor in the body of the patient S. A portion of the epithermal neutron beam contained in the neutron beam N also exhibits the above-described therapeutic effect by being reduced in speed within the body of the patient S. The epithermal neutron beam is a neutron beam having energy of less than or equal to 0.5 eV.

Here, in the present disclosure, an XYZ coordinate system is set as follows on the basis of the emission direction of the neutron beam N (refer to Figs. 3 and 5 to 8).

X-axis: an axis extending along an emission direction of the neutron beam N emitted from the neutron beam output portion 12A

Y-axis: an axis extending along a direction orthogonal to the X-axis

Z-axis: an axis extending along a direction (direction perpendicular to a floor surface F) perpendicular to the X-axis and the Y-axis

### Irradiation Room

As shown in Figs. 2 and 3, the neutron capture therapy system 100 includes two irradiation rooms 30A and 30B. The irradiation room 30A is disposed on an extension line in a direction in which the second transporting portion 16A extends. The irradiation room 30B is disposed on an extension line in a direction in which the third transporting portion 16B extends.

The neutron beamN can be extracted in a direction orthogonal to the direction in which the second transporting portion 16A or the third transporting portion 16B extends. In this case, the disposition of the irradiation room 30A is not limited to be on the extension line in the direction in which the second transporting portion 16A extends, and the irradiation room 30A may be disposed at a position corresponding to the extraction direction of the neutron beam N. The disposition of the irradiation room 30B is not limited to be on the extension line in the direction in which the third transporting portion 16B extends, and the irradiation room 30B may be disposed at a position corresponding to the extraction direction of the neutron beam N. The irradiation room 30A and the irradiation room 30B have the same configuration as each other. Therefore, in the present disclosure, the irradiation room 30A will be described, but the description of the irradiation room 30B will be omitted.

The irradiation room 30A is a room in which a patient S is placed in order to be irradiated with a neutron beam N. The size of the irradiation room 30A is, for example, 3.5 m wide x 5 m long x 3 m tall. The irradiation room 30A includes a shielding space 30S which is enclosed by a shielding wall W2, and a door D1 for allowing the treatment table 80 to pass therethrough.

As shown in Fig. 4, a cover (wall body) 31 is provided between the irradiation room 30A and the shielding body 12b. The cover 31 forms a portion of a side wall surface of the irradiation room 30A. A collimator installation portion 31a which becomes an outlet for the neutron beam N is provided in the cover 31. The collimator installation portion 31a is an opening for fitting the collimator 86 therein.

As shown in Fig. 3, the shielding wall W2 forms the shielding space 30S in which entering of a radiation ray into the irradiation room 30A from the outside thereof and releasing of the radiation ray from the inside to the outside of the irradiation room 30A are suppressed. That is, the shielding wall W2 shields the neutron beam N radiation from the inside to the outside of the irradiation room 30A. The shielding wall W2 may be integrally formed with a shielding wall W making the charged particle beam generating room 10a. The shielding wall W2 may be a concrete wall having a thickness greater than or equal to 2 m. The wall W1 partitioning the charged particle beam generating room 10a and the irradiation room 30A is provided between the charged particle beam generating room 10a and the irradiation room 30A. The wall W1 forms a portion of the shielding wall W.

The door D1 prevents the radiation rays from being released from the shielding space 30S into a communication room 40A. The door D1 is provided so as to block the entrance communicating with the communication room 40A. A driving force is imparted to the door D1 using a motor or the like on a rail provided in the irradiation room 30A to open and close the door D1 (refer to Fig. 1). When the door D1 is opened, the treatment table 80 can pass through the space between the irradiation room 30A and the communication room 40A. When the door D1 is closed, the treatment table 80 cannot pass through the space between the irradiation room 30A and the communication room 40A. The door D1 is a heavy object, and therefore, a high torque motor, a reduction gear, or the like is used as a mechanism for driving the door D1. The door D1 may have a function of informing the system of the access of an operator to the irradiation room 30A. For example, it is confirmed that the operator exits the irradiation room 30A through the door D1 closed in a state where the treatment table 80 is disposed in the irradiation room 30A.

A camera 32 is disposed in the irradiation room 30A. The camera 32 observes the state of the patient S in the irradiation room 30A. The camera 32 is disposed at a position capable of photographing the patient S in the irradiation room 30A. It is not necessary that the camera 32 obtains an image at high accuracy. The camera 32 may obtain an image capable of checking the state of the patient S. A CCD camera may be used as the camera 32, for example.

### Preparation Room

The preparation room 50A is a room for carrying out an operation required for irradiating a patient S in an irradiation room 30A with a neutron beam N. The preparation room 50A is disposed so as to be separated from the irradiation room 30A along a Y-axis direction. The preparation room 50A and the preparation room 50B have the same configuration as each other. Therefore, in the present disclosure, the preparation room 50A will be described, but the description of the preparation room 50B will be omitted.

In the preparation room 50A, for example, restraining of a patient S on the treatment table 80, or positioning of the patient S and a collimator 86 is carried out. For this reason, the preparation room 50A has a size to the extent that an operator can easily carry out the preparation around the treatment table 80 which is accommodated in the preparation room 50A.

A wall W3 partitioning the preparation room 50A and the irradiation room 30A is provided between the preparation room 50A and the irradiation room 30A. The thickness of the wall W3 is, for example, 3.2 m. That is, the preparation room 50A and the irradiation room 30A are separated from each other by 3.2 m along the Y-axis direction.

The wall W3 is provided with the communication room 40A communicating between the preparation room 50A and the irradiation room 30A. The communication room 40A is a room for moving the treatment table 80, on which the patient S is restrained, between the preparation room 50A and the irradiation room 30A. The communication room 40A has a width allowing the treatment table 80 to pass therethrough. The communication room 40A has a height allowing an operator to pass therethrough on foot. Accordingly, for example, the size of the communication room 40A is 1.5 m wide x 3.2 m long x 2.0 m tall. A door D2 is disposed between the preparation room 50A and the communication room 40A.

The preparation rooms 50A and 50B may be shielding spaces which are enclosed by a shielding wall W similarly to the irradiation rooms 30A and 30B. The preparation rooms 50A and 50B may be spaces which are not enclosed by the shielding wall W.

### Track

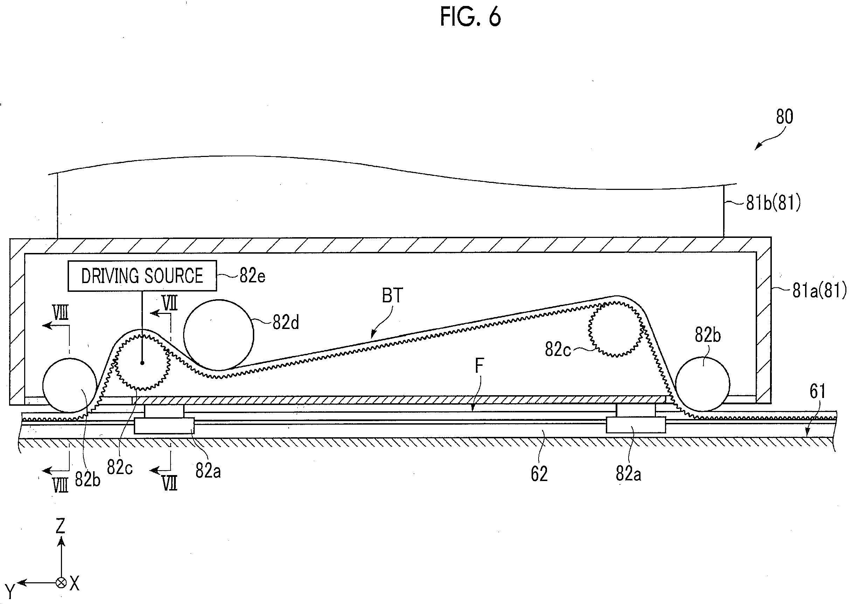

As shown in Figs. 1 and 3, the track 60 is a path consisting of a structure for moving the treatment table 80 between the irradiation room 30A and the preparation room 50A through the communication room 40A. Similarly, the track 60 extends from the irradiation room 30B to the preparation room 50B through a communication room 40B (refer to Fig. 3). For this reason, hereinafter, the track 60 extending over the irradiation room 30A, the communication room 40A, and the preparation room 50A will be described, but the description of the track 60 extending over the irradiation room 30B, communication room 40B, and the preparation room 50B will be omitted. As shown in Figs. 6 to 8, the track 60 includes a recessed groove portion 61, a guide rail 62, and support members 63.

The recessed groove portion 61 is provided on a floor surface F in the irradiation room 30A, the communication room 40A, and the preparation room 50A so as to be recessed downward from the floor surface F. For this reason, the recessed groove portion 61 extends from the irradiation room 30A to the preparation room 50A through the communication room 40A. One end of the recessed groove portion 61 is positioned in the vicinity of the neutron beam output portion 12A or the neutron beam output portion 12B. The other end of the recessed groove portion 61 is positioned inside the preparation room 50A or the preparation room 50B. That is, the recessed groove portion 61 extends in a direction leading away from the vicinity of the neutron beam output portion 12A or the neutron beam output portion 12B. In the present embodiment, the recessed groove portion 61 has a linear shape.

The guide rail 62 and the support member 63 are provided in the recessed groove portion 61 so as to extend along the recessed groove portion 61. One end of each of the guide rail 62 and the support member 63 is positioned at one end of the recessed groove portion 61. The other end of each of the guide rail 62 and the support member 63 is positioned at the other end of the recessed groove portion 61. Accordingly, similarly to the recessed groove portion 61, the guide rail 62 and the support member 63 extend from the inside to the outside of the irradiation room 30A. The tip end of the guide rail 62 and the tip end of the support member 63 are positioned at a substantially identical level and below the floor surface F. The linear distances between the tip end of the guide rail 62 and the floor surface F and between the tip end of the support member 63 and the floor surface F are substantially the same as the thickness of a belt BT to be described later.

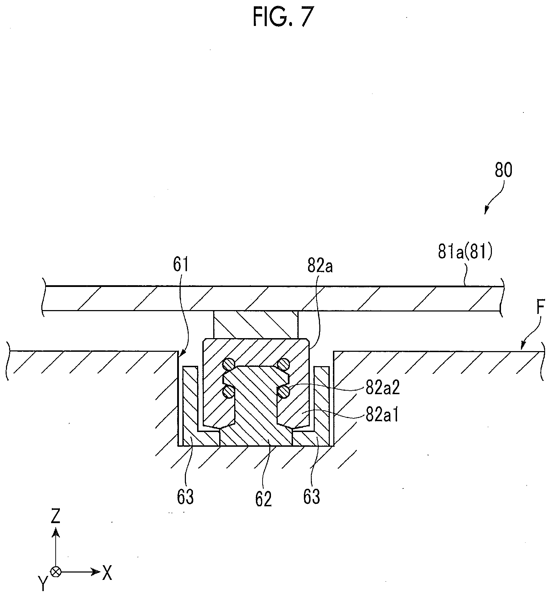

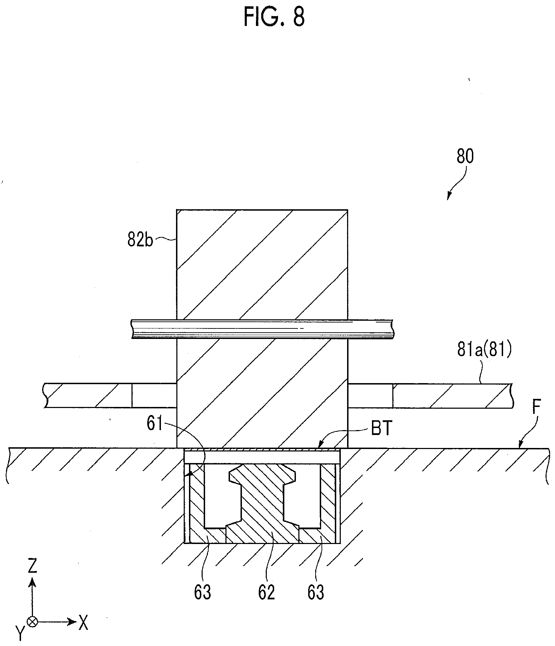

The guide rail 62 is engaged with a slider (rail engagement portion) 82a to be described later and guides the slider 82a along itself. A pair of support members 63 is disposed on both sides of the guide rail 62. The support members 63 have L-shaped cross-sectional shapes (refer to Figs. 7 and 8). The support members 63 are separated from the side surface of the guide rail 62 to the extent that the slider 82a can pass therethrough.

### Management Room

The management room 70 is a room for managing the whole process performed using the neutron capture therapy system 100. At least one manager enters the management room 70 and manages the whole process using a controller 71 which is disposed in the management room 70 and is used for operating a monitoring device and the neutron beam generating portion 10.

For example, the manager who enters the management room 70 visually confirms the state of the preparation operation in the preparation rooms 50Aand 50B, from the inside of the management room 70. The manager who enters the management room 70 operates the controller 71 to control, for example, the beam transportation path 13 so as to irradiate the target T, which corresponds to the irradiation room 30A to be irradiated with a neutron beam N, with a charged particle beam P. The manager who enters the management room 70 operates the controller 71 to control the starting and stopping of the irradiation of the target with the neutron beam N.

When carrying out the neutron capture therapy, various preparation operations (for example, PET test, or administration of boron (¹⁰B) or the like) are performed for the patient S before the patient S enters the preparation rooms 50A and 50B. Such a pre-preparation process may also be managed in the management room 70. In this case, the management room 70 manages the whole process of the neutron capture therapy including irradiation therapy using the neutron capture therapy system 100.

The management room 70 is disposed between the preparation room 50A and the preparation room 50B so as to be adjacent to the two preparation rooms 50A and 50B. The management room 70 is adjacent to the preparation room 50A in a corner portion and to the preparation room 50B in another corner portion. A window 72A for observing the inside of the preparation room 50A is disposed between the management room 70 and the preparation room 50A. A window 72B for observing the inside of the preparation room 50B is disposed between the management room 70 and the preparation room 50B. A monitor 73 for displaying an image of the camera 32 which is provided in the irradiation rooms 30A and 30B is disposed in the management room 70. The manager can check the state of the patient S in the irradiation room 30A through the image of the camera which is displayed on the monitor 73.

### Treatment Table

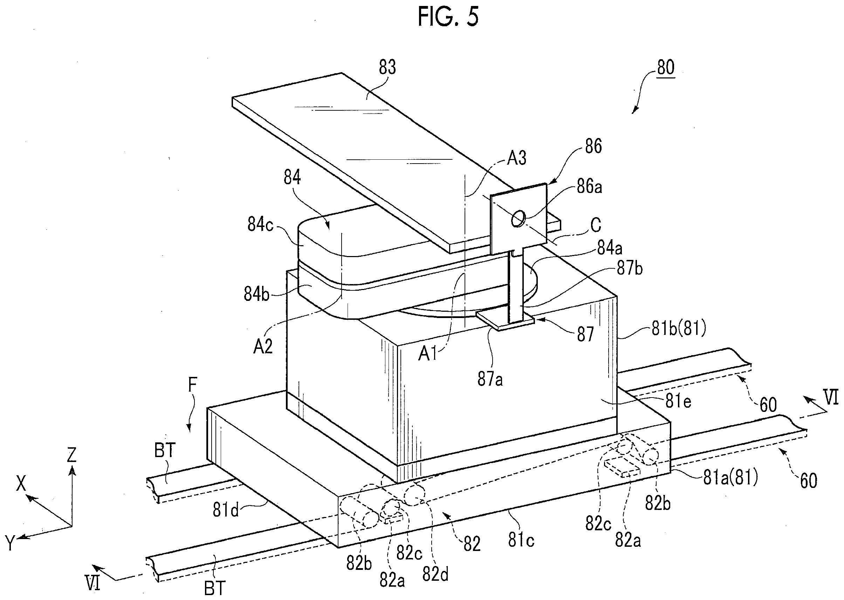

The treatment table 80 is for placing a patient S in a lying state and for restraining the patient S so as to maintain a predetermined posture in the patient S. The treatment table 80 is moved from the preparation room 50A to the irradiation room 30A while maintaining the posture of the patient S. As shown in Fig. 5, the treatment table 80 includes a base 81, a driving portion 82 for moving the base 81 on the floor surface F, a table top (placing portion) 83 for placing the patient S, a robot arm 84 for relatively moving the table top 83 on the base 81, a collimator 86 for defining an irradiation visual field of the neutron beam N, and a collimator fixing portion 87 for fixing the collimator 86 onto the base 81.

The base 81 forms a base portion of the treatment table 80. The base 81 has a foundation portion 81a and a support 81b which is disposed on the foundation portion 81a. The foundation portion 81a has a rectangular shape in a plane view, including sides 81c, 81d. For example, the side 81c is set to be longer than the side 81d. The length of at least one of the sides 81c, 81d is set to be shorter than the width of the communication rooms 40A and 40B. The support 81b has a rectangular parallelepiped shape. The lower surface of the support 81b is attached to the upper surface of the foundation portion 81a. The robot arm 84 and the collimator fixing portion 87 are disposed on the upper surface of the support 81b.

The driving portion 82 is provided on a lower surface side of the foundation portion 81a. The driving portion 82 can support the weight of all of the base 81, the robot arm 84, the table top 83, the collimator 86, the collimator fixing portion 87, and the patient S, and move them along the floor surface F. As shown in Figs. 5 and 6, the driving portion 82 has the slider 82a, a pressing roller 82b, a lifting roller 82c, a tension roller 82d, a driving source 82e, and a belt BT.

The slider 82a is set to be slidable along the guide rail 62. The slider 82a constitutes a linear motion bearing (linear guide) together with the guide rail 62. As shown in Fig. 6, the slider 82a is provided on the lower surface of the foundation portion 81a. For this reason, when the slider 82a slides along the guide rail 62, the treatment table 80 also moves along the guide rail 62.

As shown in Fig. 7, the slider 82a has a slider main body 82a1 and a rolling member 82a2 which is accommodated in the slider main body 82a1. The rolling member 82a2 has a spherical shape in the present embodiment and comes into contact with the surface of the guide rail 62. A lubricant is adhered to the surface of the rolling member 82a2. For this reason, the rolling member 82a2 rotates in response to the movement of the slider 82a and realizes accurate and smooth linear motion in the slider main body 82a1. Accordingly, some of the lubricant adhered to the surface of the rolling member 82a2 is adhered to the surface of the guide rail 62 in response to the movement of the slider main body 82a1.

As shown in Figs. 5 and 6, a pair of pressing rollers 82b is disposed in the front and rear of the foundation portion 81a. The pressing rollers 82b are attached to the foundation portion 81a so as to be rotatable. As shown in Figs. 6 and 8, the lower end of the pressing roller 82b is positioned at substantially the same level as the floor surface F. The lower surface of the pressing roller 82b comes into contact with the upper surface of the belt BT and presses the belt BT on the guide rail 62 and the support member 63.

As shown in Figs. 5 and 6, a pair of lifting rollers 82c is disposed inside the pressing rollers 82b so as to be adjacent to the pressing rollers 82b. The lifting rollers 82c are positioned above the pressing rollers 82b. The lifting rollers 82c are attached to the foundation portion 81a so as to be rotatable. The lifting rollers 82c are toothed pulleys having a surface on which recesses and projections are arranged in a circumferential direction. The lifting rollers 82c come into contact with the lower surface of the belt BT and partially lift the belt BT. In the present embodiment, any one of the pair of lifting rollers 82c is connected to the driving source 82e and is rotatably driven by the driving source 82e.

As shown in Figs. 5 and 6, the tension roller 82d is positioned between the pair of lifting rollers 82c. The tension roller 82d is attached to the foundation portion 81a so as to be rotatable. The tension roller 82d is biased downward by a biasing member which is not shown in the drawing. The lower end of the tension roller 82d comes into contact with the upper surface of the belt BT and biases the belt BT downward.

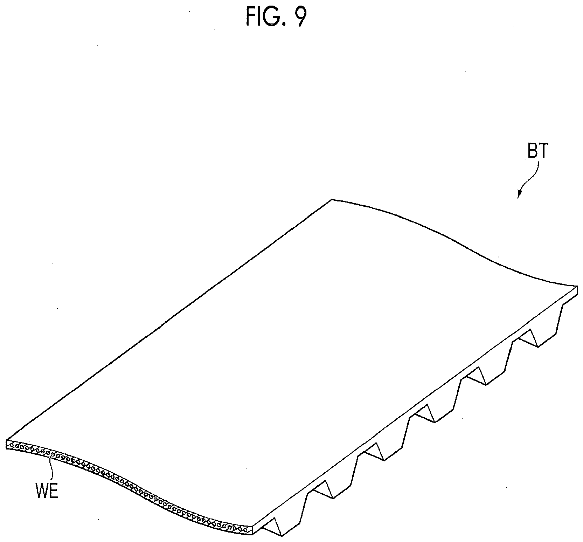

As shown in Fig. 9, the belt BT is a so-called toothed belt and has flexibility. One surface of the belt BT is a flat surface without any recesses and projections, and faces upward in the present embodiment (refer to Figs. 6 and 8). A plurality of wires WE are arranged on the flat surface side of the belt BT (refer to Fig. 9). The wires WE extend along a length direction of the belt BT. The plurality of wires WE are arranged along a width direction of the belt BT. The other surface of the belt BT is a recessed and projected surface on which recesses and projections are arranged in an extending direction of the belt BT, and faces downward in the present embodiment (refer to Figs. 6 and 8). The other surface (recessed and projected surface) of the belt BT meshes with the recesses and projections constituting the peripheral surface of the lifting roller 82c.

The belt BT is disposed in the recessed groove portion 61 and covers the upper surface of the guide rail 62 and the upper surface of the support members 63. For this reason, the lower surface of the belt BT faces the recessed groove portion 61. The portion of the belt BT which is lifted by the lifting roller 82c is positioned above the recessed groove portion 61. In contrast, an upper surface of a portion of the belt BT which is not lifted by the lifting roller 82c constitutes a substantially identical surface to the floor surface F (refer to Fig. 8). A lower surface (more specifically, a tip end of the projected portion of the belt BT) of a portion of the belt BT which is not lifted by the lifting roller 82c comes into contact with the tip end of the guide rail 62 and the tip ends of the support members 63. That is, the belt BT is supported at three points including the upper surface of the guide rail 62 and the upper surfaces of the pair of support members 63.

One end of the belt BT is fixed to one end of the recessed groove portion 61. The other end of the belt BT is fixed to the other end of the recessed groove portion 61. The width of the belt BT is set to be substantially the same as the width of the recessed groove portion 61 or slightly smaller than the width of the recessed groove portion 61. The width of the belt BT may be set to be, for example, smaller than or equal to 8 times the thickness of the belt.

The table top 83 is a flat plate having a rectangular shape. The length of the table top 83 in a longitudinal direction is set to a length (for example, about 2 m) enabling the patient S to lie down. One end of the table top 83 is attached to one end of an arm 84c so as to be rotatable around a vertical axis A3. A retaining tool (not shown) for fixing the body of the patient S is provided in the table top 83.

The robot arm 84 moves the table top 83 relatively to the base 81. That is, the robot arm 84 moves the patient S, who is restrained on the table top 83, relatively to the collimator 8 6 which is fixed to the base 81. The height from the floor surface F to the table top 83 is not particularly limited, but is preferably set to a height to the extent that restraining of the patient S on the table top 83 or the like can be easily carried out.

The robot arm 84 includes an elevating portion 84a, an arm 84b, and the arm 84c. The elevating portion 84a is disposed on an upper surface side of the base 81. One end of the arm 84b is provided on the elevating portion 84a so as to be rotatable around a vertical axis A1. One end of the arm 84c is provided on the other end of the arm 84b so as to be rotatable around the vertical axis A2. That is, the robot arm 84 has two vertical axes A1 and A2 which are separated from each other in a horizontal direction.

The robot arm 84 can move the table top 83 at a predetermined position within an XY-plane by rotating the arm 84b around the vertical axis A1 with respect to the elevating portion 84a, rotating the arm 84c around the vertical axis A2 with respect to the arm 84b, and rotating the table top 83 around the vertical axis A3 with respect to the arm 84c. That is, the robot arm 84 can rotate the body of the patient S around the vertical axis with respect to the irradiation direction of the neutron beam N. The robot arm 84 can move the table top 83 in a Z-axis direction by vertically moving the elevating portion 84a with respect to the support 81b. Accordingly, according to such a robot arm 84, it is possible to increase the degree of freedom in the posture of the patient S with respect to the collimator 86 which is fixed to the base 81.

The collimator 86 defines the irradiation range of the neutron beam N. For example, a circular opening 8 6a for defining the irradiation range is provided in the collimator 86. It is possible to irradiate a predetermined irradiation target of the patient S with a neutron beam N which passes through the opening 86a using the robot arm 84 which maintains the posture of the patient S with respect to the opening 86a at a predetermined position.

The collimator 86 is, for example, a flat plate having a rectangular shape. The outer shape of the collimator 86 corresponds to the inner shape of the collimator installation portion 31a in the irradiation room 30A. In the present disclosure, a virtual axis passing through the center (center of the opening 86a) of the irradiation field which is defined by the collimator 86 is referred to as an irradiation center axis C. The irradiation center axis C extends in an upstream and downstream direction of the neutron beam N when the treatment table 80 is disposed in the irradiation rooms 30A and 30B and the neutron beam N is emitted.

The collimator fixing portion 87 is fixed on the upper surface in the support 81b of the base 81. The collimator fixing portion 87 holds the collimator 86 at a constant position with respect to the base 81. The collimator fixing portion 87 has a horizontal piece 87a and an upright piece 87b and forms a substantially L shape. One end of the horizontal piece 87a is fixed to the support 81b and the other end thereof protrudes from a side surface 81e of the support 81b in a direction along the X-axis. One end of the upright piece 87b is fixed to the other end of the horizontal piece 87a, and the collimator 86 is attached to the other end thereof which extends in an upward direction.

The upright piece 87b is fixed to the horizontal piece 87a protruding in the direction along the X-axis further than the side surface 81e of the base 81. Therefore, the collimator 86 is held at a position protruding in the horizontal direction further than the side surface 81e of the base 81. By holding the collimator 8 6 at such a position, when attaching the collimator 86 to the collimator installation portion 31a of the cover 31, it is possible to suppress the interference of the base 81, the table top 83, and the like with the cover 31. Accordingly, it is possible to easily attach the collimator 86 to the collimator installation portion 31a.

### Flow of Treatment

Next, the flow of neutron capture therapy using a neutron capture therapy system 100 will be described.

First, predetermined pre-preparation is performed for patient S before entering a preparation room 50A (neutron capture therapy system 100). Next, the patient S and an operator enter the preparation room 50A and the operator allows the patient S to lie down on the table top 83. Next, the operator restrains the body of the patient S on the table top 83 using a restraining tool. Next, positioning of an irradiation target of the patient S and an irradiation center axis C of the collimator 86 is carried out.

Next, the treatment table 80 is moved to the irradiation room 30A. At this time, a manager in the management room 70 may determine whether to allow the treatment table into the irradiation room 30A. For example, the operator reports the completion of the operation in the preparation room 50A to the manager. When the manager who has received the report determines that the treatment table can enter the irradiation room 30A, door D2 partitioning the preparation room 50A and the communication room 40A is opened.

When the treatment table 80 moves from the preparation room 50A to the communication room 40A, the door D2 is closed. After the closing of the door D2, the door D1 partitioning the communication room 40A and the irradiation room 30A is opened. The order of opening and closing the doors D1, D2 is not limited to this order. For example, the door D1 and the door D2 may be simultaneously opened.

When the treatment table 80 moves from the communication room 40A to the inside of the irradiation room 30A, the collimator 86 is attached to the collimator installation portion 31a. Next, the door D1 is closed. Then, when the manager operates the controller 71, irradiation of a target with a neutron beam N starts and treatment of the patient S is performed. The irradiation time is, for example, about one hour. The state of the patient S during the treatment is imaged by a camera 32 which is provided inside the irradiation room 30A. An image imaged by the camera 32 is displayed on the monitor 73 in the management room 70. The manager manages the state of the patient S during treatment using the monitor 73. The manager determines whether to stop the irradiation when any abnormality is found in the patient S during the treatment.

When a predetermined irradiation time has elapsed, the controller 71 automatically stops the irradiation of the neutron beam N. Next, the treatment table 80 is moved to the preparation room 50A. Next, the operator releases the fixing of the patient S using the restraining tool in the preparation room 50A and guides the patient S to the outside of the preparation room 50A. Accordingly, the neutron capture therapy using the neutron capture therapy system 100 is finished.

### Action and Effect

According to the neutron capture therapy system 100, it is possible to selectively irradiate each of a plurality of irradiation rooms 30A and 30B with a neutron beam N. According to the neutron capture therapy system 100, it is possible to carry out the preparation operation for irradiating a patient S with a neutron beam N in each of the preparation rooms 50A and 50B, and therefore, the time required for the preparation operation in the irradiation rooms 30A and 30B is reduced. Accordingly, the ratio of the irradiation time of the patient S with the neutron beam N in the time during which the patient S is disposed in the irradiation rooms 30A and 30B is increased, and therefore, it is possible to enhance the utility efficiency of the irradiation rooms 30A and 30B.

The irradiation time in the neutron capture therapy is longer than that in radiation therapy such as X-ray therapy or proton therapy. For this reason, effectiveness in the neutron capture therapy system 100 due to the preparation operation carried out in one irradiation room 30B or the preparation room 50B in parallel with the treatment in the other irradiation room 30A, greatly contributes to the improvement of the operation efficiency of the whole system, for example. According to the neutron capture therapy system 100, a control for irradiating the irradiation rooms 30A and 30B with a neutron beam N is implemented in one management room 70, and therefore, it is possible to efficiently adjust the occupancy ratio of the neutron beam and to enhance the utilization efficiency of the accelerator 11.

In this manner, according to the neutron capture therapy system 100, it is possible to enhance the utilization efficiency of the irradiation rooms 30A and 30B and to enhance the utilization efficiency of the accelerator 11. As a result, it is possible to enhance the operation efficiency of the whole system.

The neutron capture therapy system 100 includes windows 72A and 72B capable of allowing observation of the inside of the preparation rooms 50A and 50B from the management room 70. For this reason, it is possible to observe the inside of the preparation rooms 50A and 50B from the management room 70 through the windows 72A and 72B. Accordingly, the manager can grasp the access of a patient S to each of the preparation rooms 50A and 50B and the degree of progress of the preparation operation in the preparation rooms 50A and 50B. As a result, it is possible to further enhance the operation efficiency of the neutron capture therapy system 100.

The neutron capture therapy system 100 includes a camera 32 for observing the inside of the irradiation rooms 30A and 30B from the management room 70 through the camera 32. For this reason, it is possible to observe the inside of the irradiation rooms 30A and 30B from the management room 70. Accordingly, the manager can grasp the state of a patient S in each of the irradiation rooms 30A and 30B. As a result, it is possible to enhance the safety of the neutron capture therapy system 100.

In the neutron capture therapy system 100, the treatment table 80 can move between the inside and the outside of the irradiation rooms 30A and 30B. For this reason, it is possible to carry out the preparation operation for irradiating a patient S with a neutron beam N in the outside of the irradiation rooms 30A and 30B. Accordingly, it is possible to reduce the time during which the patient S stays in the irradiation rooms 30A and 30B.

The neutron capture therapy system 100 generates neutrons by irradiating a target T with a charged particle beam P which is generated in the accelerator 11. According to such a neutron beam generating portion 10, it is possible to reduce the size of the neutron capture therapy system 100.

In the neutron capture therapy system 100, the guide rail 62 is provided inside the recessed groove portion 61. For this reason, the guide rail 62 does not protrude from the floor surface F. Accordingly, there is no concern that a person will stumble over the guide rail 62 protruding from the floor surface F. In the neutron capture therapy system 100, although the recessed groove portion 61 is provided on the floor surface F, the belt BT is provided in the recessed groove portion 61 so as to cover the upper surface of the guide rail 62. In addition, the upper surface of the portion of the belt BT which is not lifted by the lifting roller 82c constitutes a substantially identical surface to the floor surface F. For this reason, the recessed groove portion 61 is closed by the belt BT, and therefore, there is no concern that a person will stumble over the recessed groove portion 61 on the floor surface F. Accordingly, it is possible to enhance the safety thereof.

In the neutron capture therapy system 100, the lifting roller 82c partially lifts the belt BT, which is positioned inside the recessed groove portion 61, above the recessed groove portion 61. For this reason, at a site at which the belt BT is lifted by the lifting roller 82c, the guide rail 62 in the recessed groove portion 61 is exposed to the outside. The slider 82a of the treatment table 80 is engaged with the guide rail 62, which is exposed to the outside, along the guide rail 62. Accordingly, as the slider 82a moves on the guide rail 62, the portion of the belt BT corresponding to the site in which the slider 82a is engaged with the guide rail 62 is lifted by the lifting roller 82c and the remainder of the belt BT (portion of the belt BT which is not lifted by the lifting roller 82c) is provided in the recessed groove portion 61 and constitutes the an identical surface to the floor surface F. As a result, it is possible to move the treatment table 80 along the guide rail 62 while enhancing the safety thereof. Accordingly, it is possible to achieve both the movement of the treatment table 80 and the safety thereof.

The belt BT is supported at three points including the upper surface of the guide rail 62 and the upper surfaces of the pair of support members 63. For this reason, the belt BT can be securely supported by the guide rail 62 and the support members 63.

The treatment table 80 has the lifting roller 82c meshing with the recessed and projected surface of the belt BT. For this reason, power is transmitted to the belt BT meshing with the lifting roller 82c by rotating the lifting roller 82c using the driving source 82e. Accordingly, it is possible to move the treatment table 80 along the guide rail 62 using the reaction force.

The upper surface of the guide rail 62 is covered with the belt BT. For this reason, the lubricant existing on the surface of the guide rail 62 is moved only to the lower surface of the belt BT, and is not moved to the upper surface of the belt BT. The lower surface of the belt BT is not exposed to the outside, and therefore, the lubricant is prevented from being adhered to the floor surface F or the like. For this reason, the floor surface F or the like is not stained by the lubricant. Consequently, there is no concern that a person will slip on the lubricant, and therefore, it is possible to further enhance the safety thereof.

The driving portion 82 is provided in the treatment table 80, and therefore, it is possible to move the collimator 86 while maintaining the posture of a patient S. Accordingly, positioning of an irradiation target of the patient S and an irradiation center axis C of the collimator 86 can be carried out in the preparation rooms 50A and 50B in advance instead of being carried out in the irradiation rooms 30A and 30B. It is possible to reduce the operation time required for maintaining the treatment table 80 in a place with a high radiation dose by performing the maintenance of the treatment table 80 after moving the treatment table 80 to the outside of the irradiation rooms 30A and 30B.

In the treatment table 80, it is possible to match the longitudinal direction of the table top 83 with the movement direction of the treatment table 80 by rotating the table top 83 around the vertical axes A1, A2, and A3 with respect to the base 81. For this reason, the size of the doorway or the like through which the treatment table 80 passes is defined by the size of the base 81 instead of the length of the table top 83 in the longitudinal direction. Accordingly, the size of the doorway or the like through which the treatment table 80 passes is set to be as small as possible, and therefore, it is possible to further suppress the leakage of the radiation rays from the irradiation rooms 30A and 30B. That is, the widths of the communication rooms 40A and 40B through which the treatment table 80 moves are defined by the side 81c or the side 81d.

Hereinabove, the embodiment of the present invention has been described in detail, but the present invention is not limited to the above-described embodiment. For example, the number of sets of the preparation room and the irradiation room is not limited to two, and may be one or three or more.

The belt BT may not be the toothed belt. In this case, other driving sources for moving the treatment table 80 may be employed.

The pair of support members 63 may not be provided in the recessed groove portion 61. The width of the inlet portion of the recessed groove portion 61 may be set to be substantially the same as or slightly greater than the width of the belt BT, and the width of the inner portion of the recessed groove portion 61 may be set to be smaller than that of the belt BT. In this case, a step, in which the width of the recessed groove portion becomes small toward the inner side, is provided in the inlet portion of the recessed groove portion 61. Accordingly, the belt BT is supported by the step instead of the pair of support members 63.

The belt BT may not come into contact with the tip end (upper surface) of the guide rail 62 or the tip ends (upper surface) of the support members 63.

The treatment table 80 is moved along the guide rail 62 by rotatably driving the lifting roller 82c, which meshes with the belt BT, using the driving source 82e, but the method of moving the treatment table 80 is not limited thereto. For example, the driving portion 82 of the treatment table 80 may have a wheel coming into contact with the guide rail 62, and may move the treatment table 80 along the guide rail 62 by rotatably driving the wheel using the driving source 82e.

The lubricant may not adhere to the surface of the rolling member 82a2.

## Claims

1. A neutron capture therapy system (100) comprising:
an irradiation room (30A, 30B) adapted to encompass an irradiation portion;
the irradiation portion which is adapted to irradiate an irradation object with a neutron beam (N);
a recessed groove portion (61) which is provided on a floor surface (F) of the irradiation room (30A, 30B) so as to extend in a direction leading away from the vicinity of the irradiation portion;
a guide rail (62) which is provided in the recessed groove portion (61) so as to extend along the recessed groove portion (61) ;
a belt (BT) which is disposed in the recessed groove portion (61) so as to cover an upper surface of the guide rail (62); and
a treatment table (80) capable of moving along the guide rail (62),
wherein the treatment table (80) has a base (81), a placing portion (83) that is provided on the base (81) for placing the irradiation object, the system being **characterised in that** the treatment table (80) further comprises:
a belt lifting portion (82c) that is provided below the base (81), the belt lifting portion being adapted to partially lift the belt (BT), which is positioned in the recessed groove portion (61), above the recessed groove portion (61), and
wherein an upper surface of a portion of the belt (BT) which is not lifted above the recessed groove portion (61) by the belt lifting portion constitutes a substantially identical surface to the floor surface (F).

2. The neutron capture therapy system (100) according to claim 1, further comprising:
a pair of support members (63) which is positioned in the recessed groove portion (61) and on both sides of the guide rail (62), and extends along the recessed groove portion (61),
wherein the portion of the belt (BT) which is not lifted above the recessed groove portion (61) by the belt lifting portion is supported at three points including the upper surface of the guide rail (62) and upper surfaces of the pair of support members (63).

3. The neutron capture therapy system (100) according to claim 1 or 2,
wherein the guide rail (62), the recessed groove portion (61), and the belt (BT) extend from the inside of the irradiation room (30A, 30B) to the outside.

4. The neutron capture therapy system (100) according to claims 1 or 2,
wherein a recessed and projected surface on which recesses and projections are arranged in an extending direction of the belt (BT) is formed on a surface of the belt (BT) on a side facing the recessed groove portion (61),
wherein a surface of the belt lifting portion is formed with a recessed and projected portion meshing with the recessed and projected surface of the belt (BT), and
wherein the treatment table (80) further has a rail engagement portion which is engaged with the guide rail (62) and is slidable along the guide rail (62), and a driving source which rotates the belt lifting portion.

5. The neutron capture therapy system (100) according to claim 4,
wherein the rail engagement portion has a slider main body (82a1), and a rolling member (82a2) which is accommodated in the slider main body (82a1) and has a lubricant adhered to a surface thereof, and
wherein the rolling member (82a2) comes into contact with the guide rail (62).

## Patentansprüche

1. Neutronenaufnahmetherapiesystem (100), das Folgendes aufweist:
einen Bestrahlungsraum (30A, 30B), der ausgelegt ist, um einen Bestrahlungsteil aufzuweisen;
den Bestrahlungsteil, der ausgelegt ist, um ein Bestrahlungsobjekt mit einem Neutronenstrahl (N) zu bestrahlen;
einen vertieften Nutteil (61), der auf einer Bodenoberfläche (F) des Bestrahlungsraums (30A, 30B) so vorgesehen ist, dass er sich in einer Richtung erstreckt, die von der Umgebung des Bestrahlungsteils weg führt;
eine Führungsschiene (62), die in dem vertieften Nutteil (61) so vorgesehen ist, dass sie sich entlang des vertieften Nutteils (61) erstreckt;
einen Riemen (BT), der in dem vertieften Nutteil (61) so angeordnet ist, dass er eine obere Oberfläche der Führungsschiene (62) abdeckt; und
einen Behandlungstisch (80), der imstande ist, sich entlang der Führungsschiene (62) zu bewegen,
wobei der Behandlungstisch (80) eine Basis (81) und einen Anordnungsteil (83) aufweist, der auf der Basis (81) zur Anordnung des Bestrahlungsobjekts vorgesehen ist,
wobei das System **dadurch gekennzeichnet ist, dass** der Behandlungstisch (80) ferner Folgendes aufweist:
einen Riemenanhebeteil (82c), der unterhalb der Basis (81) vorgesehen ist, wobei der Riemenanhebeteil ausgelegt ist, um den Riemen (BT) teilweise anzuheben, der in dem vertieften Nutteil (61) positioniert ist, und zwar über den vertieften Nutteil (61), und
wobei eine obere Oberfläche einen Teil des Riemens (BT), der nicht über den vertieften Nutteil (61) durch den Riemenanhebeteil angehoben wird, eine im Wesentlichen identische Oberfläche zu der Bodenoberfläche (F) bildet.

2. Neutronenaufnahmetherapiesystem (100) gemäß Anspruch 1, das ferner Folgendes aufweist:
ein Paar von Stützgliedern (63), das in dem vertieften Nutteil (61) und an beiden Seiten der Führungsschiene (62) positioniert ist, und sich entlang des vertieften Nutteils (61) erstreckt,
wobei der Teil des Riemens (BT), der nicht über den vertieften Nutteil (61) durch den Riemenanhebeteil angehoben wird, an drei Punkten einschließlich der oberen Oberfläche der Führungsschiene (62) und oberen Oberflächen des Paars der Stützglieder (63) getragen wird.

3. Neutronenaufnahmetherapiesystem (100) gemäß Anspruch 1 oder 2, wobei sich die Führungsschiene (62), der vertiefte Nutteil (61) und der Riemen (BT) von dem Inneren des Bestrahlungsraums (30A, 30B) nach außen erstrecken.

4. Neutronenaufnahmetherapiesystem (100) gemäß Anspruch 1 oder 2, wobei eine vertiefte und vorragende Oberfläche, auf der Ausnehmungen bzw. Vertiefungen und Vorsprünge in einer Ausdehnungsrichtung des Riemens (BT) angeordnet sind, auf einer Oberfläche des Riemens (BT) auf einer Seite gebildet sind, die zu dem vertieften Nutteil (61) weist,
wobei eine Oberfläche des Riemenanhebeteils mit einem vertieften und vorragenden Teil gebildet ist, der sich in Eingriff mit der vertieften und vorragenden Oberfläche des Riemens (BT) befindet, und
wobei der Behandlungstisch (80) ferner einen Schieneneingriffsteil aufweist, der sich in Eingriff mit der Führungsschiene (62) befindet und entlang der Führungsschiene (62) gleitbar bzw. verschiebbar ist, sowie eine Antriebsquelle, die den Riemenanhebeteil dreht.

5. Neutronenaufnahmetherapiesystem (100) gemäß Anspruch 4, wobei der Schieneneingriffsteil einen Gleithauptkörper (82a1) und ein Rollglied (82a2) aufweist, das in dem Gleithauptkörper (82a1) untergebracht ist und ein Schmiermittel aufweist, das an einer Oberfläche von diesem anhaftet, und wobei das Rollglied (82a2) in Kontakt mit der Führungsschiene (62) kommt.

## Revendications

1. Système de thérapie par capture de neutrons (100) comprenant :
une chambre d'irradiation (30A, 30B) adaptée à englober une portion d'irradiation ;
ladite portion d'irradiation qui est adaptée à irradier un objet à irradier avec un faisceau de neutrons (N) ;
une portion de gorge en creux (61) qui est prévue sur une surface de plancher (F) de la chambre d'irradiation (30A, 30B) de façon à s'étendre dans une direction s'éloignant du voisinage de la portion d'irradiation ;
un rail de guidage (62) prévu dans la portion de gorge en creux (61) de façon à s'étendre le long de la portion de gorge en creux (61) ;
une courroie (BT) qui est disposée dans la portion de gorge en creux (61) de façon à couvrir une surface supérieure du rail de guidage (62) ; et
une table de traitement (80) pouvant se déplacer le long du rail de guidage (62),
dans lequel la table de traitement (80) comporte une base (81), une portion de placement (83) qui est prévue sur la base (81) pour placer l'objet à irradier, le système étant **caractérisé en ce que** la table de traitement (80) comprend en outre :
une portion d'élévation de courroie (82c) qui est prévue en dessous de la base (81), la portion d'élévation de courroie étant adaptée à soulever partiellement la courroie (BT), qui est positionnée dans la portion de gorge en creux (61), au-dessus de la portion de gorge en creux (61), et
dans lequel une surface supérieure d'une portion de la courroie (BT) qui n'est pas soulevée au-dessus de la portion de gorge en creux (61) par la portion d'élévation de courroie constitue une surface sensiblement identique à la surface de plancher (F).

2. Système de thérapie par capture de neutrons (100) selon la revendication 1, comprenant en outre :
une paire d'éléments supports (63) positionnés dans la portion de gorge en creux (61) et sur les deux côtés du rail de guidage (62), et qui s'étendent le long de la portion de gorge en creux (61),
dans lequel la portion de la courroie (BT) qui n'est pas soulevée au-dessus de la portion de gorge en creux (61) par la portion d'élévation de courroie est supportée en trois points comprenant la surface supérieure du rail de guidage (62) et des surfaces supérieures de la paire d'éléments supports (63).

3. Système de thérapie par capture de neutrons (100) selon la revendication 1 ou 2,
dans lequel le rail de guidage (62), la portion de gorge en creux (61) et la courroie (BT) s'étendent à partir de l'intérieur de la chambre d'irradiation (30A, 30B) vers l'extérieur.

4. Système de thérapie par capture de neutrons (100) selon les revendications 1 ou 2,
dans lequel une surface comportant des parties en creux et des parties saillantes, sur laquelle des parties en creux et des parties saillantes sont agencées dans une direction d'extension de la courroie (BT), est formée sur une surface de la courroie (BT) sur un côté tourné vers la portion de gorge en creux (61),
dans lequel une surface de la portion d'élévation de courroie est munie d'une portion en creux et en saillies qui s'engrène avec la surface en creux et en saillies de la courroie (BT), et
dans lequel la table de traitement (80) comporte en outre une portion de mise en prise de rail qui est en prise avec le rail de guidage (62) et qui peut coulisser le long du rail de guidage (62), et une source motrice qui fait tourner la portion d'élévation de courroie.

5. Système de thérapie par capture de neutrons (100) selon la revendication 4,
dans lequel la portion de mise en prise de rail comporte un corps principal de coulisseau (82a1), et un élément de roulement (82a2) qui est logé dans le corps principal de coulisseau (82a1) et comporte un lubrifiant adhérant à sa surface, et
dans lequel l'élément de roulement (82a2) vient en contact avec le rail de guidage (62).
